# EUROPÄISCHE PATENTSCHRIFT

(11) **EP 2 334 590 B1**
(45) Veröffentlichungstag und Bekanntmachung des Hinweises auf die Patenterteilung: **17.08.2016**
(21) Anmeldenummer: 10713632.7
(22) Anmeldetag: 09.04.2010
(51) Int. Cl.: C10L 3/08

(54) **ENERGIEVERSORGUNGSSYSTEM UND BETRIEBSVERFAHREN**
ENERGY SUPPLY SYSTEM AND OPERATING METHOD
SYSTÈME D'APPROVISIONNEMENT EN ÉNERGIE ET PROCÉDÉ D'EXPLOITATION

(30) Priorität: 09.04.2009 DE 102009018126
(43) Veröffentlichungstag der Anmeldung: 22.06.2011
(73) Patentinhaber: Zentrum für Sonnenenergie- und Wasserstoff-Forschung Baden-Württemberg, 70565 Stuttgart (DE)
(72) Erfinder: STÜRMER, Bernd, 70569 Stuttgart (DE); FRICK, Volkmar, 70374 Stuttgart (DE); SPECHT, Michael, 71111 Waldenbuch (DE); STERNER, Michael, 34121 Kassel (DE); HAHN, Berthold, 34125 Kassel (DE); ZUBERBÜHLER, Ulrich, 70563 Stuttgart (DE)
(74) Vertreter: Patentanwälte Ruff, Wilhelm, Beier, Dauster & Partner mbB
(86) Internationale Anmeldenummer: PCT/EP2010/054710
(87) Internationale Veröffentlichungsnummer: WO 2010/115983

(56) Entgegenhaltungen:
- EP-A1- 2 149 625
- WO-A2-2009/019159
- DE-A1-102004 030 717
- US-A1- 2003 168 864
- US-A1- 2008 245 660

## Beschreibung

Die Erfindung bezieht sich auf ein Energieversorgungssystem mit einer Stromerzeugungseinrichtung zur regenerativen Erzeugung von in ein Stromversorgungsnetz einspeisbarer elektrischer Energie und auf ein Betriebsverfahren für ein derartiges Energieversorgungssystem.

Der Einsatz erneuerbarer Energien, wie Windkraft, Solarenergie und Wasserkraft, gewinnt eine immer größere Bedeutung zur Stromerzeugung. Elektrische Energie wird typischerweise über langreichweitige, überregionale und länderübergreifend gekoppelte Stromversorgungsnetze, kurz Stromnetze bezeichnet, an eine Vielzahl von Verbrauchern geliefert. Da elektrische Energie als solche nicht in signifikantem Umfang speicherbar ist, muss die in das Stromnetz eingespeiste elektrische Leistung auf den verbraucherseitigen Leistungsbedarf, die sogenannte Last, abgestimmt werden. Die Last schwankt bekanntermaßen zeitabhängig, insbesondere je nach Tageszeit, Wochentag oder auch Jahreszeit. Klassisch wird der Lastverlauf in die drei Bereiche Grundlast, Mittellast und Spitzenlast unterteilt, und elektrische Energieerzeuger werden je nach Typ geeignet in diesen drei Lastbereichen eingesetzt. Für eine stabile und zuverlässige Stromversorgung ist ein kontinuierlicher Gleichlauf von Stromerzeugung und Stromabnahme notwendig. Eventuell auftretende Abweichungen werden durch sogenannte positive oder negative Regelenergie bzw. Regelleistung ausgeglichen. Positive Regelleistung wird benötigt, wenn das normale Stromangebot zu stark hinter dem aktuellen Strombedarf zurückbleibt, um ein unerwünschtes Absinken der Netzfrequenz und einen dadurch verursachten Zusammenbruch der Stromversorgung zu verhindern. Negative Regelleistung wird benötigt, wenn sich ein unerwarteter Überschuss an Stromerzeugungsleistung mit der Folge eines unerwünschten Frequenzanstiegs ergibt. Bei regenerativen Stromerzeugungseinrichtungen tritt die Schwierigkeit auf, dass bei bestimmten Typen, wie Windkraft und Solarenergie, die Energieerzeugungsleistung nicht zu jedem Zeitpunkt vorhanden und in bestimmter Weise steuerbar ist, sondern z.B. tageszeitlichen und witterungsbedingten Schwankungen unterliegt, die nur bedingt vorhersagbar sind.

Die Offenlegungsschrift DE 10 2009 007 567 A1 offenbart ein Verfahren zur Herstellung von Methanol durch Verwertung von Kohlendioxid aus dem Abgas fossil befeuerter Kraftwerke, Heizkraftwerke oder anderer CO₂-Emittenten, wobei das CO₂ einer Methanolsynthese mit Wasserstoff unterzogen wird, der vorzugsweise aus einer Elektrolyse mit regenerativ gewonnener elektrischer Energie erzeugt wird, insbesondere in Schwachlastphasen eines zugehörige Stromnetzes. Das synthetisierte Methanol kann in einem Methanolspeicher zwischengespeichert oder als Brennstoff einem Heiz- oder Stromerzeugungskraftwerk zugeführt werden. Eine verfahrensdurchführende Energieerzeugungsanlage beinhaltet ein Heizkraftwerk, ein Wind-, Wasser- und/oder Solarkraftwerk, eine Elektrolyseanlage, je einen Speicher für CO₂, O₂ und H₂, eine Methanolsyntheseanlage, einen Methanolspeicher und eine Steuerung, um diese Anlagenkomponenten zur Energieerzeugung in Abhängigkeit vom Strombedarf auslastungsoptimal zu steuern.

Die Offenlegungsschrift DE 43 32 789 A1 offenbart ein Verfahren zur Speicherung von Wasserstoffenergie durch Umsetzung von z.B. unter Einsatz von Solar- oder Nuklearenergie gewonnenem Wasserstoff mit Kohlendioxid in Methan oder Methanol, das dann z.B. als Treibstoff für Verkehrsmittel oder Verbrennungsanlagen genutzt werden kann.

Die Offenlegungsschrift DE 10 2004 030 717 A1 offenbart ein Verfahren und eine Vorrichtung zur Umwandlung und Speicherung von regenerativ gewonnener Energie mittels Wandlung in chemische Energie unter Verwendung von elektrischer Energie und Kohlendioxid, wobei die chemische Energie bedarfsabhängig wieder als chemische und elektrische Energie abgegeben wird. Dazu ist ein Kreislaufprozess vorgesehen, bei dem Energie aus einer geothermen bzw. regenerativen Quelle in elektrische Energie gewandelt wird, die einem Verbraucher und einer Elektrolyseeinrichtung zugeführt wird. Durch die Elektrolyse gewonnener Wasserstoff wird teils einem Verbraucher zugeführt und teils einer Synthese mit CO₂ aus einem CO₂-Speicher zu einem Kohlenwasserstoff und einem Alkohol unterzogen. Der Kohlenwasserstoff, z.B. Methan, wird in einem zugehörigen Speicher gespeichert und teils einem Verbraucher, teils einem Verbrennungsheizprozess zugeführt, dem andererseits Sauerstoff aus der Elektrolyse zugeführt wird. Durch einen thermodynamischen Prozess erzeugt der Verbrennungsheizprozess elektrische Energie, welche teils dem elektrischen Verbraucher und teils dem Elektrolyseprozess zugeführt wird. Im Verbrennungsheizprozess erzeugtes CO₂ wird ebenso wie CO₂ gespeichert, das aus einem CO₂-Rückgewinnungsprozess stammt, der mit CO₂ aus dem Kohlenwasserstoff-Verbraucher gespeist wird.

Die Offenlegungsschrift WO 2009/019159 A2 offenbart ein Energieverteilungsverfahren, mit dem ein Strom oder Lasten verteilendes Stromnetz stabil betrieben werden soll, wobei regenerativ erzeugte Energie zumindest in merklichem Anteil zur Erzeugung von Wasserstoff verwendet wird, Kohlendioxid aus anderen Kraftwerken oder einem Endlager zusammen mit dem Wasserstoff in einer Hydrieranlage hydriert wird und ein dabei erzeugter, gasförmiger, brennbarer Kohlenwasserstoff in einem Kraftwerk zur Generierung von elektrischem Strom verwertet wird, wobei Kohlenstoff in einem Kreislauf bewegt wird.

Für Gasversorgungsnetze ist es bekannt, dass dem üblicherweise in einem Versorgungsgebiet verteilten Gas, dem sogenannten Grundgas, wie Erdgas, ein Zusatzgas und/oder ein Austauschgas beigemischt werden kann. Unter Zusatzgasen werden Gasgemische verstanden, die sich in der Zusammensetzung und den brenntechnischen Kenndaten vom Grundgas wesentlich unterscheiden, jedoch in Mischung mit dem Grundgas ein zum Grundgas gleichartiges Brennverhalten aufweisen und dem Grundgas in begrenzter Menge zugesetzt werden dürfen. Unter Austauschgas werden Gasgemische verstanden, die trotz ihrer vom Grundgas abweichenden Zusammensetzung und gegebenenfalls abweichenden brenntechnischen Kenndaten bei gleichem Gasdruck ein gleichartiges Brennverhalten wie das Grundgas aufweisen und das Grundgas bei Bedarf vollständig ersetzen können. Daneben können noch sogenannte Konditionierungsgase beigemischt werden. Für die entsprechenden Festlegungen und Klassifizierungen bezüglich Zusatzgas, Austauschgas und Gase zur Konditionierung kann auf die diesbezügliche Fachliteratur verwiesen werden, siehe beispielsweise DVGW Technische Regel Arbeitsblatt G260: Gasbeschaffenheit, Wirtschafts- und Verlagsgesellschaft Gas und Wasser mbH, Bonn, Januar 2000; DVGW Technische Regel Arbeitsblatt G262: Nutzung von Gasen aus regenerativen Quellen in der öffentlichen Gasversorgung, Wirtschafts- und Verlagsgesellschaft Gas und Wasser mbH, Bonn, November 2004; Gaswärme-Institut e.V. Essen et al.: Analyse und Bewertung der Nutzungsmöglichkeiten von Biomasse, Band 4, 2005; und die von der Fachagentur nachwachsende Rohstoffe e.V. herausgegebene Studie: Einspeisung von Biogas in das Erdgasnetz, Leipzig, 2006, ISBN 3-00-018346-9, siehe dort insbesondere die Seiten 68 bis 70.

Der Erfindung liegt als technisches Problem die Bereitstellung eines Energieversorgungssystems der eingangs genannten Art und eines hierfür geeigneten Betriebsverfahrens zugrunde, mit denen sich regenerativ erzeugte elektrische Energie optimiert nutzen lässt, um eine stabile und zuverlässige Stromversorgung mit hohem Anteil an Strom aus erneuerbaren Energien auch dann zu gewährleisten, wenn regenerative Stromerzeuger mit merklich schwankender Erzeugungsleistung verwendet werden, wie Windkraft- und/oder Photovoltaikanlagen.

Die Erfindung löst dieses Problem durch die Bereitstellung eines Energieversorgungssystems mit den Merkmalen des Anspruchs 1 und eines zugehörigen Betriebsverfahrens mit den Merkmalen des Anspruchs 11.

Das erfindungsgemäße Energieversorgungssystem beinhaltet zusätzlich zur regenerativen Stromerzeugungseinrichtung eine Wasserstofferzeugungseinrichtung, die Wasserstoff unter Verwendung der regenerativ erzeugten elektrischen Energie erzeugen kann, und eine Methanisierungseinrichtung, die den erzeugten Wasserstoff mit einem zugeführten Kohlenoxidgas, vorzugsweise Kohlendioxid oder ein Synthesegas, in ein methanhaltiges Gas umwandeln kann. Weiter beinhaltet das Energieversorgungssystem eine Gasbereitstellungseinrichtung, mit der ein Gas in der Qualität eines in ein Gasversorgungsnetz einspeisbaren Zusatz- oder eines Austauschgases unter Verwendung des methanhaltigen Gases aus der Methanisierungseinrichtung und/oder des Wasserstoffs aus der Wasserstofferzeugungseinrichtung bereitgestellt werden kann. Mit diesen Anlagenkomponenten ist das Energieversorgungssystem charakteristischerweise in der Lage, aus regenerativ erzeugter elektrischer Energie in großem Maßstab ein Zusatz- oder Austauschgas bereitzustellen, das in ein Gasversorgungsnetz eingespeist und dort gespeichert oder anderweitig genutzt werden kann. Für die genannten Systemkomponenten sind an sich bekannte Anlagen einsetzbar, wie eine Elektrolyseeinheit zur Wasserstofferzeugung und ein üblicher Methanisierungsreaktor.

Speziell ist die Gasbereitstellungseinrichtung dafür ausgelegt, das Gas in einer variabel vorgebbaren Zusatz-/Austauschgasqualität bereitzustellen, wozu sie bei Bedarf über entsprechende Gasaufbereitungsmittel verfügt. Durch diese spezielle Auslegung der Gasbereitstellungseinrichtung kann optimal situationsangepasst das Gas in der Qualität eines Zusatzgases bereitet werden, um dieses z.B. in begrenzter Menge in ein Gasversorgungsnetz einzuspeisen, oder in der Qualität eines Austauschgases bereitet werden, um dieses z.B. als vollwertigen Grundgasersatz in ein Gasversorgungsnetz einzuspeisen. Die Gasaufbereitung in einer Zusatzgasqualität erfordert typischerweise niedrigeren Energieaufwand als die Bereitung eines Austauschgases, dafür besteht für die Einspeisung des Austauschgases in das Gasversorgungsnetz keine Beschränkung, da es das Grundgas gegebenenfalls vollständig ersetzen kann. Es versteht sich, dass die Gasbereitstellungseinrichtung einschließlich ihrer zugehörigen Steuerungsmittel je nach Bedarf als eigenständige Anlagenkomponente realisiert oder ganz oder teilweise in die Methanisierungseinrichtung und/oder die Wasserstofferzeugungseinrichtung und/oder eine zentrale Anlagensteuerung integriert sein kann. In jedem Fall lässt sich damit in sehr vorteilhafter Weise das unter Verwendung regenerativer elektrischer Energie erzeugte Gas je nach Bedarf unterschiedlich und variabel als Zusatz- oder Austauschgas bereitstellen und z.B. zur Einspeisung in ein Gasversorgungsnetz nutzen.

In einer vorteilhaften Weiterbildung der Erfindung beinhaltet das Energieversorgungssystem eine Verstromungseinrichtung, die elektrische Energie unter Verwendung von Gas aus dem Gasversorgungsnetz erzeugen kann. Hierfür eignet sich z.B. eine hocheffiziente Gas- und Dampf-Kraftwerkanlage, abgekürzt GuD-Kraftwerk, oder eine andere Art der Methanverstromung, wie Gasturbinen oder mit Methan befeuerte Blockheizkraftwerke mit Gas-Otto-Motoren, für Methan adaptierte Motoren oder Brennstoffzellen. Stellvertretend für alle Möglichkeiten der Methanverstromung wird im Weiteren primär das GuD-Kraftwerk genannt.

Das Energieversorgungssystem ermöglicht damit eine Verlagerung von regenerativer fluktuierender Stromerzeugung und somit eine erhöhte Nutzbarkeit von regenerativen Stromerzeugern mit typbedingt fluktuierender Erzeugungsleistung unter Beibehaltung der erforderlichen Netzstabilität eines davon gespeisten Stromnetzes. In Zeiträumen, in denen regenerativ erzeugter Strom z.B. aus Windkraft- und/oder Photovoltaikanlagen aus Gründen der Netzstabilität oder anderen technischen und wirtschaftlichen Gründen nicht in das Stromnetz eingespeist werden kann, lässt er sich zur Erzeugung von Wasserstoff nutzen, der mit Kohlenoxidgas mittels Methanisierung in ein Zusatz- oder Austauschgas umgesetzt wird, welches in das Gasversorgungsnetz eingespeist und dort gespeichert und genutzt werden kann, insbesondere auch zur Rückverstromung in Zeiträumen mit hoher residualer Last des Stromnetzes, d.h. mit hoher Differenz zwischen Leistungsbedarf des Stromnetzes und Leistungsangebot durch die regenerative Stromerzeugungseinrichtung.

Die Erfindung löst somit in sehr effizienter Weise die Probleme der mangelnden Speicherbarkeit hoher elektrischer Energiemengen, wie sie für öffentliche Stromnetze benötigt werden, des z.B. tageszeitlich, saisonal und witterungsbedingt fluktuierenden Leistungsangebots wichtiger regenerativer Stromerzeuger und der erforderlichen Netzstabilität des Stromnetzes. Das erfindungsgemäße Energieversorgungssystem ermöglicht eine Vernetzung bzw. Kopplung von Strom- und Gasversorgungsnetz, die eine hohe Auslastung regenerativer Stromerzeuger durch Nutzung der vorhandenen herkömmlichen Gasspeicherkapazitäten im Gasversorgungsnetz erlaubt und zudem durch entsprechendes Last- und Erzeugungsmanagement hochwertige, regenerative positive und negative Regelenergie bzw. gespeicherten regenerativen Strom für das Stromnetz bereitstellen kann.

In vorteilhafter Ausgestaltung der Erfindung umfasst die Stromerzeugungseinrichtung insbesondere eine oder mehrere Windkraftanlagen und/oder eine oder mehrere Photovoltaikanlagen und/oder ein oder mehrere Geothermiekraftwerke und/oder ein oder mehrere Biomassekraftwerke und/oder ein oder mehrere Wasserkraftwerke und/oder ein oder mehrere Solarthermiekraftwerke. Diesen Anlagetypen wird in Zukunft eine stark zunehmende Bedeutung für die Versorgung mit elektrischer Energie zuerkannt. Ihre spezifische Einbindung in das erfindungsgemäße Energieversorgungssystem erlaubt einen hohen Nutzungsgrad derselben bei aufrechterhaltener Stromnetzstabilität trotz des zeitlich stark fluktuierenden Leistungsangebots einiger dieser regenerativen Stromerzeuger und der zeitlich schwankenden Stromnachfrage.

In einer Weiterbildung der Erfindung weist das Energieversorgungssystem Mittel zum Zuführen von Kohlendioxid zu der Methanisierungseinrichtung aus einem Kohlendioxid abgebenden Kraftwerk auf. Dies ermöglicht die Nutzung und Einbindung von in einem Kraftwerk anfallendem Kohlendioxid über die Methanisierungseinrichtung in den Energiekreislauf des Energieversorgungssystems.

In einer Weiterbildung der Erfindung beinhaltet das Energieversorgungssystem Mittel zum Zuführen eines kohlendioxidhaltigen Gases zu der Methanisierungseinrichtung aus einer Biogasanlage, wie Biogas oder daraus abgetrenntes Kohlendioxid. Dadurch kann vorteilhaft die regenerative Biomassenutzung einer üblichen Biogasanlage in das erfindungsgemäße Energieversorgungssystem eingebunden sein.

In einer Weiterbildung der Erfindung sind erfindungsgemäß Mittel zur Wärmeübertragung von der Methanisierungseinrichtung zu einer Biogasanlage vorgesehen. Auf diese Weise lässt sich Abwärme der Methanisierungseinrichtung in der Biogasanlage nutzen.

In einer Weiterbildung der Erfindung sind Mittel zum Zuführen eines von einer Biogasanlage gewonnenen Zusatz- oder Austauschgases zur Gasbereitstellungseinrichtung oder zum Gasversorgungsnetz vorgesehen, d.h. die Biogasanlage liefert in diesem Fall auch einen direkten Beitrag zur Speisung des Gasversorgungsnetzes.

In einer weiteren vorteilhaften Weiterbildung der Erfindung beinhaltet das Energieversorgungssystem Mittel zum Zuführen von kohlenoxidhaltigem Synthesegas aus einer Biomasse-Vergasungsanlage zur Methanisierungseinrichtung. Durch entsprechende Auslegung der Methanisierungseinrichtung kann das Synthesegas zusammen mit dem Wasserstoff in das methanhaltige Zusatz- oder Austauschgas umgesetzt werden.

In einer Weiterbildung der Erfindung beinhaltet das Energieversorgungssystem eine ORC-Anlage (Organic-Rankine-Cycle-Anlage) oder andere Wärmenutzungsanlage zur Erzeugung elektrischer Energie unter Verwendung von Abwärme der Methanisierungseinrichtung, so dass diese Abwärme direkt zur Stromerzeugung genutzt werden kann.

In einer Weiterbildung der Erfindung umfasst das Energieversorgungssystem eine Tankstelleneinrichtung, durch die entsprechende Fahrzeuge mit von der Wasserstofferzeugungseinrichtung erzeugtem Wasserstoff und/oder mit von der regenerativen Stromerzeugungseinrichtung erzeugter elektrischer Energie und/oder mit von der Gasbereitstellungseinrichtung geliefertem Zusatz- oder Austauschgas versorgt werden können, wobei je nach Anwendungsfall und Bedarf an der Tankstelleneinrichtung alle drei oder nur ein Teil dieser Energieträger zur Fahrzeugbetankung bereitgestellt sein können.

In einer Weiterbildung der Erfindung umfasst das Energieversorgungssystem ein Stromversorgungsnetz, an das die regenerative Stromerzeugungseinrichtung und die Wasserstofferzeugungseinrichtung sowie die Verstromungseinrichtung angekoppelt sind, und/oder ein Gasversorgungsnetz, an das die Methanisierungseinrichtung und die Verstromungseinrichtung angekoppelt sind. Damit sind das Stromversorgungsnetz und/oder das Gasversorgungsnetz in das erfindungsgemäße Energieversorgungssystem eingebunden, vorzugsweise beide in miteinander spezifisch gekoppelter Form. In einer Ausgestaltung dieses Erfindungsaspekts umfasst die Energieversorgungseinrichtung einen an das Gasversorgungsnetz angekoppelten Gasspeicher. Hierbei kann es sich um einen für diesen Zweck an sich bekannten Gasspeicher handeln, der in der Lage ist, das von der Methanisierungseinrichtung gelieferte Erdgassubstitut mit hoher Kapazität zwischenzuspeichern.

In einer Weiterbildung der Erfindung umfasst das Energieversorgungssystem eine Steuerungseinrichtung, die dafür eingerichtet ist, die Leistung der Wasserstofferzeugungseinrichtung und/oder der Verstromungseinrichtung in Abhängigkeit von einem zeitabhängigen Leistungsbedarf des Stromversorgungsnetzes variabel einzustellen. Dies bildet die Grundlage für ein vorteilhaftes und effizientes Last- und Erzeugungsmanagement des Systems und insbesondere des Stromversorgungsnetzes.

In einer Ausgestaltung dieses Erfindungsaspektes ist die Steuerungseinrichtung dafür ausgelegt, in Zeiträumen mit erhöhtem Erzeugungsleistungsbedarf des Stromnetzes eine verringerte Aufnahmeleistung der Wasserstofferzeugungseinrichtung einzustellen und/oder eine erhöhte Einspeiseleistung der Verstromungseinrichtung einzustellen und/oder in Zeiträumen mit verringertem Erzeugungsleistungsbedarf die Aufnahmeleistung der Wasserstofferzeugungseinrichtung zu steigern und/oder die Einspeiseleistung der Verstromungseinrichtung zu reduzieren. Mit anderen Worten kann die Steuerungseinrichtung positive und negative Regelenergie zur Gewährleistung der Netzstabilität des Stromnetzes unter Verwendung der regenerativ erzeugten und als Wasserstoff und/oder Erdgassubstitut im Gasversorgungsnetz rückverstrombar zwischengespeicherten elektrischen Energie bereitstellen. Dies wird durch einen Betrieb des Systems mit dem erfindungsgemäßen Verfahren ermöglicht.

In einer anderen Ausgestaltung dieses Erfindungsaspektes ist die Steuerungseinrichtung dafür ausgelegt, die Leistung der Wasserstofferzeugungseinrichtung und/oder der Verstromungseinrichtung in Abhängigkeit von einem prognostizierten Leistungsbedarfsprofil des Stromversorgungsnetzes und/oder eines prognostizierten Stromerzeugungsleistungsprofils der regenerativen Stromerzeugungseinrichtung einzustellen. Diese Auslegung der Steuerungseinrichtung ermöglicht einen vorausschauenden Betrieb des Energieversorgungssystems unter dem Gesichtspunkt der geforderten Stromnetzstabilität und unter Berücksichtigung des gegebenenfalls fluktuierenden Leistungsangebots der regenerativen Stromerzeugungseinrichtung und des schwankenden Strombedarfs. Dazu lässt sich das Energieversorgungssystem durch das entsprechend weitergebildete erfindungsgemäße Betriebsverfahren betreiben.

In einer Weiterbildung der Erfindung wird das Zusatz- oder Austauschgas wählbar in einer von mehreren vorgebbaren Qualitätsstufen bereitgestellt. Dies ermöglicht eine optimale Anpassung des Betriebs der Methanisierungseinrichtung und/oder der Wasserstofferzeugungseinrichtung an die Anforderungen, die z.B. seitens des Gasversorgungsnetzes an das Zusatz-/Austauschgas gestellt werden. Wenn vom Gasversorgungsnetz erlaubt, kann mit geringerem Aufwand ein Zusatzgas einer niedrigeren Qualitätsstufe bereitgestellt werden, bei höheren Anforderungen kann mit typischerweise etwas höherem Aufwand ein Zusatz- oder Austauschgas höherer Qualitätsstufe bereitgestellt werden.

Vorteilhafte Ausführungsformen der Erfindung sind in den Zeichnungen dargestellt und werden nachfolgend beschrieben. Hierbei zeigen:
- Fig. 1: ein schematisches Blockdiagramm eines Energieversorgungssystems mit regenerativen Stromerzeugern, Wasserstofferzeugungseinrichtung, Methanisierungseinrichtung und Verstromungseinrichtung unter entsprechender Kopplung eines Stromversorgungsnetzes und eines Gasversorgungsnetzes,
- Fig. 2: eine Blockdiagrammdarstellung entsprechend Figur 1 mit detaillierterer Darstellung von möglichen Betriebsvarianten,
- Fig. 3: eine Blockdiagrammdarstellung entsprechend Figur 1 für eine Variante mit ORC-Anlage,
- Fig. 4: eine Blockdiagrammdarstellung entsprechend Figur 1 für eine Variante mit Kohlendioxidnutzung aus einem Kraftwerk zur Verstromung fossiler Energieträger,
- Fig. 5: eine Blockdiagrammdarstellung entsprechend Figur 1 für eine Variante mit Nutzung von abgetrenntem Kohlendioxid aus einer Biogasanlage,
- Fig. 6: eine Blockdiagrammdarstellung entsprechend Figur 1 für eine Variante mit Nutzung eines Biogases aus einer Biogasanlage als Kohlendioxidquelle für die Methanisierung,
- Fig. 7: eine Blockdiagrammdarstellung entsprechend Figur 1 für eine Variante mit Nutzung eines Synthesegases aus einem Biomasse-Vergasungsreaktor als Kohlenoxidquelle und
- Fig. 8: eine Blockdiagrammdarstellung entsprechend Figur 1 für eine Variante mit zusätzlicher Tankstelleneinrichtung zur Fahrzeugbetankung mit Wasserstoff, elektrischer Energie und/oder Erdgassubstitut.

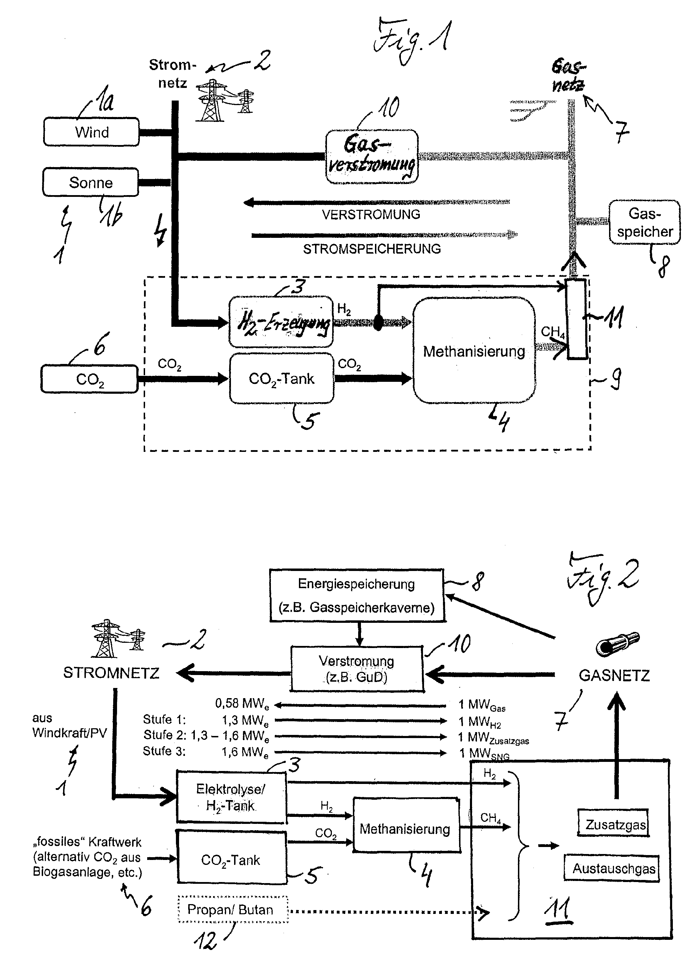

Fig. 1 zeigt schematisch ein Energieversorgungssystem, das eine Speicherung regenerativ erzeugter elektrischer Energie als Zusatz- oder Austauschgas mit bedarfsweiser Rückverstromung ermöglicht. Das Energieversorgungssystem beinhaltet eine regenerative Stromerzeugungseinrichtung 1, die im gezeigten Beispiel Windkraftanlagen 1 a und Photovoltaikanlagen 1 b umfasst und elektrische Energie aus erneuerbaren Energien zur Einspeisung in ein Stromnetz 2 erzeugt, insbesondere ein öffentliches Stromnetz. Weiter beinhaltet das Energieversorgungssystem eine an das Stromnetz 2 als Verbraucher angekoppelte Wasserstofferzeugungseinrichtung 3 und eine dieser nachgeschaltete Methanisierungseinrichtung 4, die den in der Wasserstofferzeugungseinrichtung 3 unter Verwendung elektrischer Energie aus dem Stromnetz 2 erzeugten Wasserstoff zusammen mit zugeführtem Kohlenoxidgas, z.B. Kohlendioxid (CO₂), in Methangas bzw. ein methanreiches Gas umsetzt. Das Kohlendioxid entstammt einem Kohlendioxid-Tank 5, der von einer Kohlendioxidquelle 6 gespeist wird. Von einer Gasbereitstellungseinrichtung 11 wird das von der Methanisierungseinrichtung 4 erzeugte Gas dazu verwendet, ein Zusatz- oder Austauschgas mit vorgebbarer Gasqualität bereitzustellen und in ein Gasversorgungsnetz 7, abgekürzt Gasnetz, einzuspeisen, an das ein Gasspeicher 8 angekoppelt ist. Optional kann ein gewisser Teil des von der Wasserstofferzeugungseinrichtung 3 erzeugten Wasserstoffs (H₂) auch direkt unter Umgehung der Methanisierungseinrichtung 4 der Gasbereitstellungseinrichtung 11 zur Bereitstellung des Zusatz- oder Austauschgases zugeführt werden.

Die Wasserstofferzeugungseinrichtung 3, die Methanisierungseinrichtung 4, der CO₂-Tank 5 und die Gasbereitstellungseinrichtung 11 bilden somit einen stromwandelnden Systemteil 9, der Strom aus dem Stromnetz 2 und insbesondere Strom aus der regenerativen Stromerzeugungseinrichtung 1 in ein Zusatz- oder Austauschgas umwandelt, das leicht in großen Mengen im Gasnetz 7 gespeichert werden kann, bei dem es sich insbesondere um ein übliches Gasnetz mit den an sich bekannten Gasspeicherkapazitäten handeln kann. Invers zum Systemteil 9 beinhaltet das Energieversorgungssystem eine Verstromungseinrichtung 10 zur Verstromung von aus dem Gasnetz 7 entnommenem Gas und Einspeisung der dadurch erzeugten elektrischen Energie in das Stromnetz 2. Insbesondere kann dadurch das unter Nutzung der regenerativ erzeugten elektrischen Energie gewonnene Zusatz- oder Austauschgas rückverstromt werden. Der stromwandelnde Systemteil 9 kann hierbei örtlich getrennt vom Gasspeicher 8 und von der Verstromungseinrichtung 10 sowie auch von der CO₂-Quelle 6 installiert sein.

Fig. 2 veranschaulicht etwas detaillierter einige mögliche Varianten der Realisierung eines solchen Energieversorgungssystems und des Betriebs desselben. Wie angedeutet, kann die Wasserstofferzeugungseinrichtung 3 z.B. durch eine Elektrolyseeinheit herkömmlicher Bauart und einen angekoppelten Wasserstofftank gebildet sein. Als mögliche CO₂-Quelle 6 sind stellvertretend eine Biogasanlage oder ein CO₂ emittierendes Kraftwerk zur Verstromung eines fossilen Energieträgers angeführt. Die Verstromungseinrichtung 10 kann z.B. durch ein GuD-Kraftwerk oder durch ein Blockheizkraftwerk, abgekürzt BHKW, realisiert sein. Der Gasspeicher 8 kann z.B. übliche geologische Poren- und/oder Kavernenspeicher beinhalten.

Durch entsprechende Auslegung der Gasbereitstellungseinrichtung 11 kann je nach Bedarf variabel bzw. wahlweise Zusatz- oder Austauschgas in unterschiedlichen Gasqualitäten unter Verwendung des in der Wasserstofferzeugungseinrichtung 3 erzeugten Wasserstoffs und/oder des in der Methanisierungseinrichtung 4 erzeugten methanhaltigen Gases bereitgestellt und in das Gasnetz 7 eingespeist werden. In einem in Fig. 2 angedeuteten Ausführungsbeispiel ist eine Bereitstellung von Zusatz-/Austauschgas wahlweise in einer von drei unterschiedlichen Gasqualitäten vorgesehen. Als eine erste Stufe ist die Zugabe von praktisch reinem Wasserstoff aus der Wasserstofferzeugungseinrichtung 3 als Zusatzgas zum Grundgas des Gasnetzes 7 derart vorgesehen, dass hinter einer entsprechenden Gasmischstelle noch ein einspeisefähiges Gas resultiert, das für das Gasnetz 7 vorgeschriebene Einspeiserichtlinien erfüllt. Für eine Wasserstoff-Erzeugungsleistung von 1 MW werden dazu bei Einsatz einer hocheffizienten Elektrolyse ca. 1,3 MW elektrische Leistung benötigt. In einer zweiten Stufe wird ein Zusatzgas eingespeist, das neben einem noch möglichst hohen Wasserstoffanteil einen geringfügigen Methananteil aus der Methanisierungseinrichtung 4 derart enthält, dass nach der Gasmischstelle wiederum ein Gas resultiert, das die Einspeiserichtlinien erfüllt. Der Wasserstoffanteil kann der Gasbereitstellungseinrichtung 11 direkt durch die Wasserstofferzeugungseinrichtung 3 und/oder über die Methanisierungseinrichtung 4 zugeführt werden. Letzteres kann dadurch erreicht werden, dass der Wasserstoff aus der Wasserstofferzeugungseinrichtung 3 derart durch die Methanisierungseinrichtung 4 geführt und der Betrieb der Methanisierungseinrichtung 4 derart eingestellt wird, dass die Methanisierungseinrichtung 4 nur einen Teil des zugeführten Wasserstoffs in Methan umsetzt und den übrigen zugeführten Wasserstoff zusammen mit dem erzeugten Methan abgibt. Für die Gewinnung von 1 MW Leistung an derartigem Zusatzgas werden ca. 1,3 MW bis 1,6 MW elektrische Leistung benötigt. In einer dritten Stufe wird ein Austauschgas, auch als Erdgassubstitut bezeichnet, eingespeist, wozu der Wasserstoffanteil des Austauschgases auf unter 5 Vol.-% und der CO₂-Anteil auf unter 6 Vol-% begrenzt sind. Für 1 MW Leistung an derartigem Ergassubstitut (substitute natural gas; SNG) werden ca. 1,6 MW elektrische Leistung benötigt. Dabei entsprechen 1,6 MW elektrische Leistung ca. 0,2 Tonnen an pro Stunde der Methanisierungseinrichtung 4 zuzuführendem Kohlendioxid.

Je nach Anwendungsfall und Bedarf kann jede der drei Stufen dahingehend modifiziert bzw. aufgespalten werden, dass zusätzlich zum Zusatz- /Austauschgas optional ein Konditioniergas zugemischt wird, z.B. um gewünschte brenntechnische Kennwerte für das Gas hinter der Einspeisestelle einzustellen. Hierfür sind die üblichen Konditioniergase verwendbar, insbesondere C₂₋₄-Kohlenwasserstoffe, wie Propan und/oder Butan, speziell z.B. LPG (Liquefied Petroleum Gas), und/oder DME (Dimethylether), die aus einer entsprechenden Kohlenwasserstoffquelle 12 zugeführt werden, Luft und/oder Stickstoff. Eine weitere Qualitätsstufenverfeinerung kann bei Bedarf durch spezifische quantitative Vorgaben für den beizumischenden Wasserstoff- bzw. Methananteil vorgesehen werden. Mit jeder Qualitätsstufe nimmt der Energieaufwand für die Bereitstellung des entsprechenden Gases zu, dafür aber auch die Gasqualität. Während Zusatzgas im Allgemeinen nur in begrenzter Menge dem Grundgas im Gasnetz beigemischt werden kann, eignet sich Austauschgas als Erdgassubstitut für einen zum Grundgas äquivalenten Einsatz, und im Gegensatz zu Zusatzgas hat der Gasnetzbetreiber meist sogar die Verpflichtung, Austauschgas zur Einspeisung in sein Gasnetz anzunehmen.

Es versteht sich, dass die Gasbereitstellungseinrichtung 11 mit den zur variablen Bereitstellung des Zusatz-/Austauschgases in verschiedenen Gasqualitäten erforderlichen Komponenten ausgerüstet ist, einschließlich entsprechender Steuerungsmittel, welche die Beimischung der verschiedenen genannten Gasbestandteile und bei Bedarf auch den Betrieb der diese Gasbestandteile liefernden Anlagenkomponenten geeignet steuern bzw. regeln. Insbesondere verfügt die Gasbereitstellungseinrichtung 11 über entsprechende Mittel zur Erkennung der Zusammensetzung des im Gasnetz bereits vorhandenen Grundgases, oder es wird ihr diese Information zugeführt, damit sie über die einzustellende Gasqualität des Zusatz-/Austauschgases entscheiden kann. Alternativ kann ihr die Information über die jeweils aktuell gewünschte Zusatz-/Austauschgasqualität von außen zugeführt werden.

Mit der erläuterten Systemauslegung lässt sich ein vergleichsweise hoher Stromspeicherwirkungsgrad erzielen, der je nach Anforderungen der Gaskonditionierung zwischen ca. 35% und 45% liegt. Das vorliegende System ermöglicht eine deutlich verbesserte Ausnutzung des Potentials von Windenergie und anderen regenerativen Stromerzeugungsarten, wobei in kürzester Zeit hochwertige Regelenergie für das Stromnetz bereitgestellt werden kann, indem die Wasserstofferzeugungseinrichtung 3 und die Verstromungseinrichtung 10 entsprechend abhängig vom Leistungsbedarf des Stromnetzes 2 gesteuert werden. Die Einspeisung von erzeugtem Zusatz-/Austauschgas in ein vorhandenes Gasnetz ermöglicht eine standortunabhängige Verstromung ohne die Problematik einer lokalen Wasserstoffspeicherung. Neben dieser Stromspeicherungs- und Stromlastregelungsfunktionalitäten bietet dieses Energieversorgungssystem zudem den Vorteil einer Nutzung von anderweitig anfallendem und ansonsten ungenutzt bleibendem Kohlendioxid.

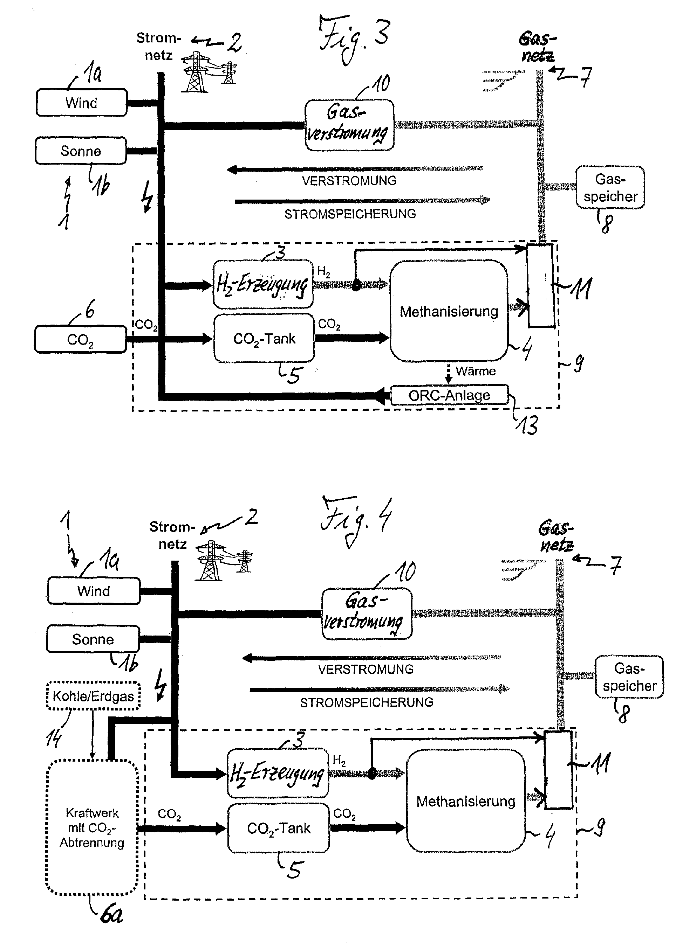

Nachfolgend werden unter Bezugnahme auf die Fig. 3 bis 8 einige vorteilhafte Realisierungsvarianten des Energieversorgungssystems näher erläutert, wobei der Übersichtlichkeit halber für gleiche oder funktionell äquivalente Komponenten jeweils gleiche Bezugszeichen verwendet sind.

Die in Fig. 3 gezeigte Systemvariante beinhaltet neben den Basiskomponenten gemäß Fig. 1 eine ORC-Anlage 13, in welcher Abwärme aus der Methanisierungseinrichtung 4 zur zusätzlichen Erzeugung elektrischer Energie genutzt wird, die in das Stromnetz 2 eingespeist wird. Alternativ kann zu diesem Zweck anstelle der ORC-Anlage 13 eine andere herkömmliche Wärmenutzungsanlage dienen.

Die in Fig. 4 gezeigte Systemvariante nutzt als Kohlendioxidquelle ein herkömmliches Kraftwerk 6a, das elektrische Energie durch Verstromung eines fossilen Energieträgers 14, wie Kohle und/oder Erdgas, erzeugt und in das Stromnetz 2 einspeist und über eine CO₂-Abtrennung verfügt. Das abgetrennte CO₂ wird im CO₂-Tank 5 zwischengespeichert.

Bei dem CO₂-lieferenden Kraftwerk kann es sich z.B. um ein solches vom sogenannten "carbon-capture"-Typ handeln. Der Transport des Kohlendioxids vom Kraftwerk 6a zum CO₂-Tank 5 kann beispielsweise in verflüssigter Form über eine Pipeline oder über Straßen-, Schienen- oder Schiffstransport erfolgen.

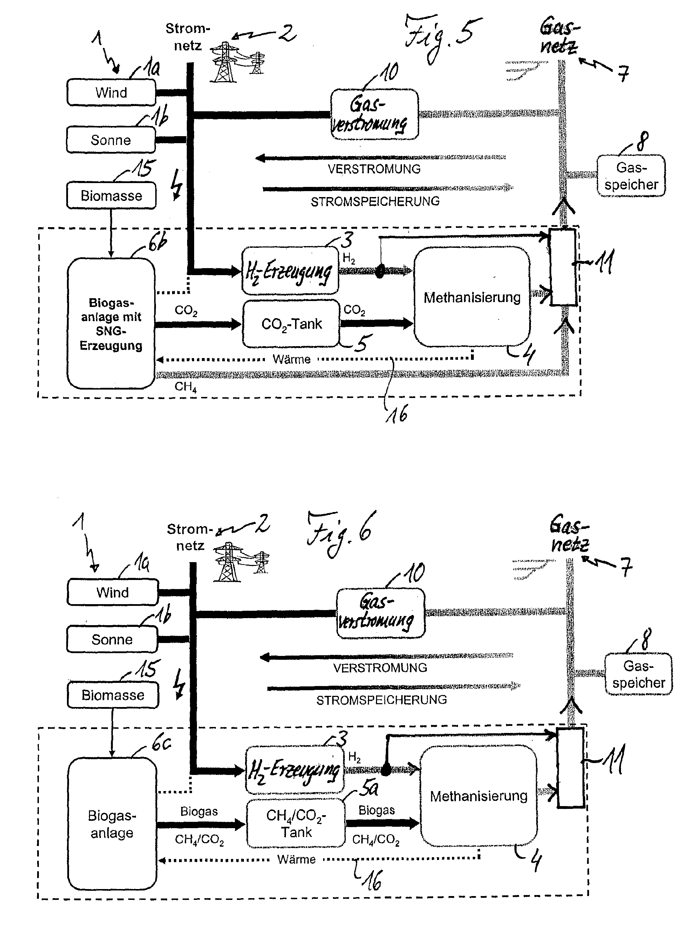

Die in Fig. 5 gezeigte Systemvariante nutzt als CO₂-Quelle eine Biogasanlage 6b, die zur Erzeugung eines Biogases aus zugeführter Biomasse und zur CO₂-Abtrennung aus dem erzeugten Biogas ausgelegt ist. Durch die CO₂-Abtrennung wird aus dem Biogas ein einspeisefähiges, methanhaltiges Zusatzgas oder Austauschgas erhalten, das von der Biogasanlage 6b der Gasbereitstellungseinrichtung 11 zugeführt oder im letztgenannten Fall als Erdgassubstitut auch direkt in das Gasnetz 7 eingespeist werden kann. Über einen optionalen Wärmetransportpfad 16 kann Abwärme aus der Methanisierungseinrichtung 4 zur Biogasanlage 6b zwecks dortiger Nutzung transportiert werden. Alternativ kann diese Wärme auch anderweitig genutzt, z.B. in ein Wärmenetz eingespeist, werden.

Die in Fig. 6 gezeigte Systemvariante nutzt als CO₂-Quelle eine Biogasanlage 6c wie die Systemvariante von Fig. 5, in diesem Fall jedoch ohne CO₂-Abtrennung aus dem erzeugten Biogas. Das CO₂-haltige Biogas wird in einem entsprechenden Biogastank 5a zwischengespeichert, der hier als Kohlendioxid-Tank fungiert, und von diesem der Methanisierungseinrichtung 4 zugeführt. Die Methanisierungseinrichtung 4 ist in diesem Fall geeignet zur Umsetzung des Biogases mit Wasserstoff ausgelegt, um das gewünschte Zusatz-/Austauschgas zu erzeugen.

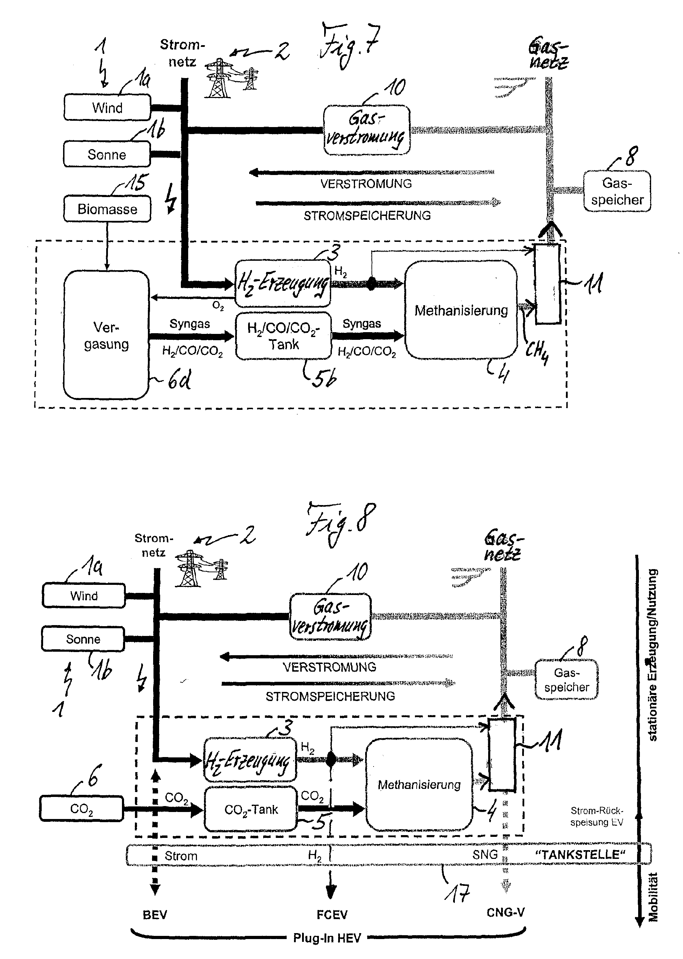

Die in Fig. 7 gezeigte Systemvariante beinhaltet einen herkömmlichen Vergasungsreaktor 6d zur Vergasung von Biomasse 15, wozu in der Wasserstofferzeugungseinrichtung 3 durch den Elektrolyseprozess gewonnener Sauerstoff dem Vergasungsreaktor 6d zugeführt werden kann. Dort kann der Sauerstoff für eine autotherme Prozessführung der Vergasungsreaktion genutzt werden. Das vom Vergasungsreaktor 6d erzeugte, kohlenoxidhaltige Synthesegas wird in einem entsprechenden Synthesegastank 5b zwischengespeichert und von dort der Methanisierungseinrichtung 4 zugeführt, bei Bedarf nach entsprechender Gasreinigung. Die Methanisierungseinrichtung 4 ist in diesem Fall darauf ausgelegt, sowohl das Kohlendioxid als auch das Kohlenmonoxid, die im Synthesegas neben Wasserstoff enthalten sind, in Methan umzusetzen, wobei zusätzlich der Wasserstoff aus der Wasserstofferzeugungseinrichtung 3 genutzt wird.

Fig. 8 veranschaulicht eine Systemvariante, die zusätzlich mit einer Tankstelleneinrichtung 17 ausgerüstet ist, die eine Fahrzeugbetankung wahlweise mit elektrischer Energie, Wasserstoff oder Zusatzgas bzw. bevorzugt Erdgassubstitut ermöglicht. So lassen sich Elektrofahrzeuge (battery electric vehicle; BEV) an einem entsprechenden Stromanschluss auftanken, der an das Stromnetz 2 gekoppelt ist. An einem Wasserstoffanschluss lassen sich mit Wasserstoff betriebene Fahrzeuge, z.B. Brennstoffzellenfahrzeuge (fuel cell electric vehicle; FCEV), mit Wasserstoff betanken, und an einem Gasanschluss lassen sich gasbetriebene Fahrzeuge (compressed natural gas vehicle; CNG-V) mit Erdgassubstitut betanken. Alternativ kann die Gasbereitstellungseinrichtung 11 in diesem Fall auch zur Bereitstellung eines der DIN-Norm 51624 entsprechenden Fahrzeugkraftstoffgases ausgelegt sein, das in seiner Gasqualität noch über die Austauschgas-Mindestqualität hinausgeht. Dies umfasst die Möglichkeit" ein unter der Bezeichnung "Hythane" bekanntes H₂/CH₄-Kraftstoffgas bereitzustellen. In jedem Fall lassen sich alle drei Arten der Energiebetankung auf die regenerativ erzeugte elektrische Energie zurückführen, d.h. die Fahrzeuge werden vollständig durch erneuerbare Energien gespeist. Über einen entsprechenden Kombinationsanschluss (plug-in HEV) können auch Hybridelektrofahrzeuge betankt werden, die mit mehreren dieser drei Energieträger betrieben werden können.

Es versteht sich, dass die zu den einzelnen gezeigten Systemvarianten der Fig. 3 bis 8 jeweils erwähnten Komponenten in beliebiger Weise kombinierbar sind. So kann z.B. die ORC-Anlage 13 von Fig. 3 auch bei den anderen Systemvarianten installiert sein, und statt nur einer Kohlenoxidquelle können in entsprechenden weiteren Systemvarianten mehrere der erwähnten Kohlenoxidquellen 6, 6a bis 6d parallel vorgesehen sein.

Die Methanisierungseinrichtung 4 ist für eine jeweils optimale Umsetzung des zugeführten Kohlenoxidgases mit dem zugeführten Wasserstoff ausgelegt. Mit Ausnahme der Systemvariante von Fig. 7 wird CO₂ als Kohlenoxidgas zugeführt. In diesen Fällen erfolgt die Umsetzung über die insgesamt stark exotherme Kohlendioxid-Hydrierungsreaktion

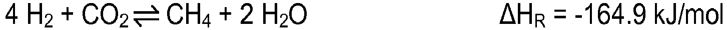

Diese ist über die endotherme Retro-Shiftreaktion 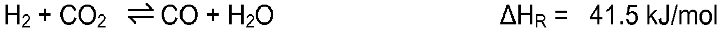 mit der CO-Methanisierungsreaktion 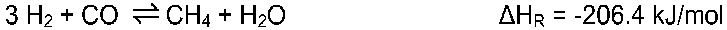 gekoppelt. Eine solche Methanisierung eines weitestgehend aus CO₂ bestehenden Gases ohne höheren CO-Anteil bedarf einer geeigneten Prozessführung und Reaktorauslegung. Im Fall des Einsatzes eines Biomasse-Vergasungsreaktors, wie bei der Systemvariante von Fig. 7, ist die Methanisierungseinrichtung 4 auf die Methanisierung des erzeugten Synthesegases ausgelegt.

In allen erwähnten Varianten beinhaltet das Energieversorgungssystem eine Steuerungseinrichtung zur Steuerung bzw. Regelung des Gesamtsystems, die in einer an sich herkömmlichen Weise als System- bzw. Anlagensteuerung implementiert sein kann und daher hier nicht näher gezeigt ist und nachfolgend nur mit ihren hier interessierenden, charakteristischen Steuerungs-/Regelungsfunktionalitäten erläutert wird.

Die in der Steuerungseinrichtung implementierte Systemsteuerung basiert zunächst auf herkömmlichen Stromnetzsteuerungsmaßnahmen, wie der Einteilung in Grundlast, Mittellast und Spitzenlast des Stromnetzes 2. Zusätzlich werden die regenerativen Stromerzeuger in solche vom fluktuierenden Typ, wie Wind- und Photovoltaikanlagen, und solche vom steuerbaren Typ unterschieden, wie Biogasanlagen, Wasserkraftanlagen, Geothermiekraftwerke und Meeresenergiekraftwerke. Für einen stabilen Betrieb des Stromnetzes 2 ist sogenannte Regelleistung bzw. Leistungsreserve notwendig, um unerwartete Ungleichgewichte zwischen Bedarf und Erzeugung elektrischer Energie auszugleichen und dabei die Netzfrequenz in jedem Fall innerhalb eines vorgegebenen Toleranzbereichs zu halten. Positive Regelleistung wird benötigt, wenn die Stromerzeugungsleistung unerwartet abfällt und/oder die Last, d.h. Verbrauchsleistung, unerwartet ansteigt. Negative Regelleistung wird z.B. benötigt, wenn leistungsstarke Verbraucher kurzzeitig ausfallen. Mit zunehmendem Anteil regenerativer Stromerzeuger mit fluktuierendem Stromangebot steigt der Bedarf an Regelleistung.

Die für das vorliegende Energieversorgungssystem ausgelegte Steuerungseinrichtung ermittelt zunächst als Vorausabschätzung die sogenannte residuale Last als denjenigen Strombedarf, der voraussichtlich nicht durch die fluktuierenden regenerativen Stromerzeuger, wie Windenergie- und Photovoltaikanlagen, gedeckt werden kann. Für diese Prognose können standardisierte Lastprofile des zu erwartenden Strombedarfs einerseits und Vorhersagen über die zu erwartende Einspeisungsleistung der regenerativen Stromerzeugungseinrichtung z.B. unter Berücksichtigung von Wetterdaten verwendet werden. Die residuale Last wird über den Tagesverlauf hinweg in verschiedene Zeitzonen mit Zeiträumen hoher residualer Last, niedriger residualer Last und mittlerer residualer Last unterteilt. In den Zeiträumen mit niedriger residualer Last steuert die Steuerungseinrichtung die betroffenen Systemkomponenten so, dass regenerativ erzeugte elektrische Energie als Erdgassubstitut gespeichert wird, d.h. die Wasserstofferzeugungseinrichtung 3 und die Methanisierungseinrichtung 4 werden aktiviert bzw. in ihrer Erzeugungsleistung angehoben. In Zeiträumen hoher residualer Last bleiben die Wasserstofferzeugungseinrichtung 3 und die Methanisierungseinrichtung 4 deaktiviert oder in einem Zustand reduzierter Leistung, und die Gasverstromungseinrichtung 10 wird aktiviert, um zwischengespeichertes Erdgassubstitut rückzuverstromen und dadurch zusätzlich elektrische Energie bereitzustellen. In den Zwischenzeiten kann je nach Bedarf und Anwendungsfall die regenerativ erzeugte elektrische Energie direkt genutzt oder in Teilauslastung der Wasserstofferzeugungseinrichtung 3 und der Methanisierungseinrichtung 4 partiell zur Erzeugung von Erdgassubstitut herangezogen werden. Mit diesen Steuerungs- bzw. Regelungsmaßnahmen lassen sich die tageszeitlichen Schwankungen der residualen Last signifikant reduzieren und dadurch die Einspeisung aus fluktuierenden regenerativen Stromerzeugern verstetigen. Mit diesen Maßnahmen wird gewährleistet, dass die Netzstabilität und Versorgungssicherheit des Stromnetzes 2 durch den vermehrten Einsatz von fluktuierenden regenerativen elektrischen Energieerzeugern nicht beeinträchtigt werden.

Zur Bereitstellung positiver Regelleistung wird die Gasverstromungseinrichtung 10 aktiviert. Alternativ oder zusätzlich wird die Wasserstofferzeugungseinrichtung 3 temporär abgeschaltet oder in ihrer Leistung reduziert. Zur Bereitstellung negativer Regelleistung wird die Leistungsabnahme der Wasserstofferzeugungseinrichtung 3 hochgefahren.

Der stromwandelnde Systemteil 9 kann je nach Bedarf und Anwendungsfall unterschiedlich betrieben werden. Eine Möglichkeit besteht darin, in einem Dauerbetrieb einen gewissen Teil der regenerativ erzeugten elektrischen Energie permanent in Erdgassubstitut zu wandeln. Eine andere Möglichkeit besteht in einem Blockbetrieb, bei dem die Wasserstofferzeugungseinrichtung nur in Zeiträumen niedriger residualer Last aktiv ist. Im Dauerbetrieb wird ein Teil der Leistung der regenerativen Stromerzeuger für die Wasserstofferzeugung vorgehalten. Das kontinuierlich erzeugte Erdgassubstitut kann als Regelenergie dem Gasnetz 7 entnommen werden, und es kann sowohl positive als auch negative Regelenergie im Sekundenbereich bereitgestellt werden. Dies ist einer hohen Auslastung der Methanisierungseinrichtung 4 und damit einer hohen Wirtschaftlichkeit des Gesamtsystems förderlich. Im Blockbetrieb kann beispielsweise im Fall einer Windkraftanlage bei hohem Windaufkommen und geringem Strombedarf die überschüssige Windenergie in Erdgassubstitut gewandelt und im Gasnetz 7 zwischengespeichert werden. In Zeiträumen mit geringem Windaufkommen und hoher Stromnachfrage kann die so zwischengespeicherte Energie wieder rückverstromt werden.

Wie aus den obigen Ausführungen ersichtlich ist, ermöglicht das erfinderungemäße Energieversorgungssystem eine Kopplung eines Strom- und eines Gasnetzes mit der Möglichkeit einer Speicherung großer Mengen an regenerativ erzeugter elektrischer Energie in Form von Erdgassubstitut im Gasnetz. Dabei lassen sich Stromspeicherwirkungsgrade von bis zu ca. 45% erzielen. Als weitere Option kann ansonsten ungenutztes Kohlendioxid genutzt werden. Das Energieversorgungssystem lässt sich so betreiben, dass trotz vermehrtem Einsatz von regenerativen Stromerzeugern mit merklich fluktuierendem Leistungsangebot die Netzstabilität und Versorgungssicherheit des Stromnetzes nicht beeinträchtigt werden.

Alternativ zu den obigen Varianten mit Rückverstromung eignet sich das erfindungsgemäße Energieversorgungssystem bei Bedarf auch für eine reine Methanerzeugung. Das regenerativ synthetisierte Methan wird in diesem Fall statt einer Rückverstromung einer anderen üblichen Nutzung zugeführt, so dass die Verstromungseinrichtung dann nicht zwingend erforderlich ist. In dieser Variante eignet sich das System z.B. als Stand-alone-System zur reinen Methanerzeugung in entlegenen Regionen.

Wie die obigen Erläuterungen weiter deutlich machen, ermöglicht die Erfindung die Nutzung nicht nur von Wasserstoff und CO₂, sondern auch eines durch Methanisierung gewonnenen methanhaltigen Gases sowie eines z.B. durch Biomassevergasung gewonnenen CO-haltigen Gases als Einsatzgase für die Bereitstellung eines Gases mit einer Gasqualität, die es als in ein Gasnetz einspeisefähiges Zusatz-/Austauschgas qualifiziert. Das Zusatz-/Austauschgas kann CO₂-neutral bereitgestellt werden, wobei das CO₂ insbesondere aus Luft, einem Biogas und/oder einem Biomasse-Vergasungsgas stammen kann. Zur Bereitstellung von Regelleistung bzw. zur Regelleistungsvorhaltung ist ein intermittierender bzw. geregelter Betrieb des Elektrolyseprozesses möglich, wobei der Methanisierungsprozess nicht in gleichem Maß intermittierend ablaufen braucht, was Vorteile hinsichtlich Standzeit und Rentabilität des Methanisierungsprozesses hat. Durch die Möglichkeiten der Gaszwischenspeicherung und eines intelligenten Regelkonzeptes ist eine Entkopplung von typisch kürzerer Elektrolyselaufzeit, z.B. weniger als 2000 h/Jahr, und typisch längerer Methanisierungslaufzeit, z.B. von bis etwa 8760 h/Jahr, erzielbar. Für eine bedarfsgerechte Betriebsführung des Gesamtsystems können bei Bedarf auch Wetterdaten bzw. Wetterprognosen berücksichtigt werden, um die zu erwartende Leistung an regenerativer Stromerzeugung abzuschätzen.

## Patentansprüche

1. Energieversorgungssystem mit
- einer Stromerzeugungseinrichtung (1) zur regenerativen Erzeugung von in ein Stromversorgungsnetz (2) einspeisbarer elektrischer Energie,
- einer Wasserstofferzeugungseinrichtung (3) zur Wasserstofferzeugung unter Verwendung von elektrischer Energie der regenerativen Stromerzeugungseinrichtung,
- einer Methanisierungseinrichtung (4) zur Umwandlung von durch die Wasserstofferzeugungseinrichtung erzeugtem Wasserstoff und einem zugeführten Kohlenoxidgas in ein methanhaltiges Gas und
- einer Gasbereitstellungseinrichtung (11) mit Gasaufbereitungsmitteln zur Bereitung und Bereitstellung eines Zusatzgases oder Austauschgases in einer variabel vorgebbaren, zur Einspeisung in ein Gasversorgungsnetz geeigneten Zusatz-/Austauschgasqualität unter Verwendung entsprechend variabler Anteile des methanhaltigen Gases aus der Methanisierungseinrichtung und des Wasserstoffs aus der Wasserstofferzeugungseinrichtung und mit zugehörigen Steuerungsmitteln, die dafür ausgelegt sind, die Zusammensetzung eines im Gasversorgungsnetz bereits vorhandenen Grundgases zu erkennen oder eine diesbezüglich zugeführte Information zu empfangen und in Abhängigkeit davon über die einzustellende Zusatz-/Austauschgasqualität zu entscheiden und die Beimischung der verschiedenen Gasbestandteile für die Bereitung des Zusatz- oder Austauschgases in der betreffenden Gasqualität variabel zu steuern.

2. Energieversorgungssystem nach Anspruch 1, **gekennzeichnet durch** eine Verstromungseinrichtung (10) zur Erzeugung elektrischer Energie unter Verwendung von Gas aus einem Gasversorgungsnetz.

3. Energieversorgungssystem nach Anspruch 1 oder 2, **dadurch gekennzeichnet, dass** die regenerative Stromerzeugungseinrichtung eine oder mehrere Windkraftanlagen (1a) und/oder eine oder mehrere Photovoltaikanlagen (1b) und/oder ein oder mehrere Geothermiekraftwerke und/oder ein oder mehrere Biomassekraftwerke und/oder ein oder mehrere Wasserkraftwerke und/oder ein oder mehrere Solarthermiekraftwerke umfasst.

4. Energieversorgungssystem nach einem der Ansprüche 1 bis 3, **gekennzeichnet durch**
- Mittel zum Zuführen von Kohlendioxid zu der Methanisierungseinrichtung aus einem Kohlendioxid abgebenden Kraftwerk und/oder
- Mittel zum Zuführen eines kohlendioxidhaltigen Gases zu der Methanisierungseinrichtung aus einer Biogasanlage und/oder
- Mittel zur Zufuhr von kohlenoxidhaltigem Synthesegas aus einer Biomasse-Vergasungsanlage zur Methanisierungseinrichtung und/oder
- Mittel zur Wärmeübertragung von der Methanisierungseinrichtung zur Biogasanlage und/oder
- Mittel zur Zufuhr eines von der Biogasanlage gewonnenen Zusatz- oder Austauschgases zur Gasbereitstellungseinrichtung oder zum Gasversorgungsnetz.

5. Energieversorgungssystem nach einem der Ansprüche 1 bis 4, **gekennzeichnet durch**
- eine ORC-Anlage (13) oder andere Wärmenutzungsanlage zur Erzeugung elektrischer Energie unter Verwendung von Abwärme der Methanisierungseinrichtung und/oder
- eine Tankstelleneinrichtung (17) zur Versorgung von Fahrzeugen mit von der Wasserstofferzeugungseinrichtung erzeugtem Wasserstoff und/oder mit von der regenerativen Stromerzeugungseinrichtung erzeugter elektrischer Energie und/oder mit von der Gasbereitstellungseinrichtung bereitgestelltem Zusatz- oder Austauschgas.

6. Energieversorgungssystem nach einem der Ansprüche 2 bis 5, **gekennzeichnet durch** ein Stromversorgungsnetz (2), an das die regenerative Stromerzeugungseinrichtung, die Wasserstofferzeugungseinrichtung und die Verstromungseinrichtung angekoppelt sind, und/oder ein Gasversorgungsnetz (7), an das die Gasbereitstellungseinrichtung und die Verstromungseinrichtung angekoppelt sind.

7. Energieversorgungssystem nach Anspruch 6, **gekennzeichnet durch** einen Gasspeicher (8), der an das Gasversorgungsnetz angekoppelt ist.

8. Energieversorgungssystem nach Anspruch 6 oder 7, **gekennzeichnet durch** eine Steuerungseinrichtung, die dafür eingerichtet ist, die Aufnahmeleistung der Wasserstofferzeugungseinrichtung und/oder die Einspeiseleistung der Verstromungseinrichtung in Abhängigkeit von einem zeitabhängigen Leistungsbedarf des Stromnetzes variabel einzustellen.

9. Energieversorgungssystem nach Anspruch 8, **dadurch gekennzeichnet, dass**
- die Steuerungseinrichtung dafür ausgelegt ist, in Zeiträumen mit erhöhtem Erzeugungsleistungsbedarf des Stromversorgungsnetzes eine reduzierte Abnahmeleistung der Wasserstofferzeugungseinrichtung und/oder eine erhöhte Einspeiseleistung der Verstromungseinrichtung einzustellen und/oder in Zeiträumen mit verringertem Erzeugungsleistungsbedarf des Stromversorgungsnetzes eine erhöhte Abnahmeleistung der Wasserstofferzeugungseinrichtung und/oder ein reduzierte Einspeiseleistung der Verstromungseinrichtung einzustellen und/oder
- die Steuerungseinrichtung dafür ausgelegt ist, die Abnahmeleistung der Wasserstofferzeugungseinrichtung und/oder die Einspeiseleistung der Verstromungseinrichtung in Abhängigkeit von einem prognostizierten Leistungsbedarfsprofil des Stromversorgungsnetzes und/oder eines prognostizierten Stromerzeugungsleistungsprofils der regenerativen Stromerzeugungseinrichtung einzustellen.

10. Energieversorgungssystem nach einem der Ansprüche 1 bis 9, **dadurch gekennzeichnet, dass** die Gasbereitstellungseinrichtung dafür ausgelegt ist, das Zusatz- oder Austauschgas wählbar in einer von mehreren vorgebbaren Qualitätsstufen bereitzustellen.

11. Verfahren zum Betrieb eines Energieversorgungssystems nach einem der Ansprüche 1 bis 10, wobei die Abnahmeleistung der Wasserstofferzeugungseinrichtung und/oder die Einspeiseleistung der Verstromungseinrichtung in Abhängigkeit von einem zeitabhängigen Leistungsbedarf des Stromversorgungsnetzes variabel eingestellt wird und/oder wobei das Zusatzgas oder Austauschgas in einer variabel vorgebbaren, zur Einspeisung in ein Gasversorgungsnetz geeigneten Zusatz-/Austauschgasqualität unter Verwendung des methanhaltigen Gases aus der Methanisierungseinrichtung und des Wasserstoffs aus der Wasserstofferzeugungseinrichtung in Abhängigkeit von der erkannten oder zugeführten Grundgaszusammensetzung im Gasversorgungsnetz bereitgestellt wird.

12. Verfahren nach Anspruch 11, **dadurch gekennzeichnet, dass** in Zeiträumen mit erhöhtem Leistungsbedarf des Stromversorgungsnetzes eine verringerte Abnahmeleistung der Wasserstofferzeugungseinrichtung und/oder eine erhöhte Einspeiseleistung der Verstromungseinrichtung eingestellt wird und/oder in Zeiträumen mit verringertem Leistungsbedarf des Stromversorgungsnetzes eine erhöhte Abnahmeleistung der Wasserstofferzeugungseinrichtung und/oder eine reduzierte Einspeiseleistung der Verstromungseinrichtung eingestellt wird.

13. Verfahren nach Anspruch 11 oder 12, **dadurch gekennzeichnet, dass** die Abnahmeleistung der Wasserstofferzeugungseinrichtung und/oder die Einspeiseleistung der Verstromungseinrichtung in Abhängigkeit von einem prognostizierten Leistungsbedarfsprofil des Stromversorgungsnetzes und/oder eines prognostizierten Stromerzeugungsleistungsprofils der Stromerzeugungseinrichtung eingestellt wird.

14. Verfahren nach einem der Ansprüche 11 bis 13, **dadurch gekennzeichnet, dass** das Zusatz- oder Austauschgas wahlweise in einer von mehreren vorgebbaren Qualitätsstufen bereitgestellt wird.

## Claims

1. An energy supply system, comprising
- an electricity generating device (1) for regeneratively generating electrical energy feedable into an electricity supply grid (2),
- a hydrogen generating device (3) for generating hydrogen using electrical energy from the regenerative electricity generating device,
- a methanation device (4) for converting hydrogen generated by the hydrogen generating device and a supplied carbon oxide gas into a gas containing methane; and
- a gas providing device (11) having gas preparation means for preparing and providing one of a supplementary gas and a replacement gas in a variably specifiable supplementary/replacement gas quality suitable for feeding into a gas supply grid by using correspondingly variable amounts of the gas containing methane from the methanation device and the hydrogen from the hydrogen generating device, and having corresponding control means configured to detect the composition of a base gas already present in the gas grid or to receive an information correspondingly supplied, and to decide in dependence thereof on the gas quality to be set for the supplementary/replacement gas, and to variably control the admixture of the various gas constituents for preparing the supplementary/replacement gas in the decided gas quality.

2. The energy supply system as claimed in Claim 1, **characterized by** a gas-into-electricity conversion device (10) for generating electrical energy using gas from a gas supply grid.

3. The energy supply system as claimed in Claim 1 or 2, **characterized in that** the regenerative electricity generating device comprises one or more wind power plants (1a), and/or one or more photovoltaic power plants (lb), and/or one or more geothermal power plants, and/or one or more biomass power plants, and/or one or more hydroelectric power plants, and/or one or more solar thermal power plants.

4. The energy supply system as claimed in any of Claims 1 to 3, **characterized by**
- means for supplying carbon dioxide to the methanation device from a power plant outputting carbon dioxide, and/or
- means for supplying a gas containing carbon oxide to the methanation device from a biogas plant, and/or
- means for supplying a synthesis gas containing carbon oxide from a biomass gasification installation to the methanation device, and/or
- means for the transfer of heat from the methanation device to the biogas plant, and/or
- means for supplying a supplementary or replacement gas obtained from the biogas plant to the gas providing device or to the gas supply grid.

5. The energy supply system as claimed in any of Claims 1 to 4, **characterized by**
- an ORC unit (13) or other heat utilization installation for generating electrical energy using waste heat from the methanation device, and/or
- a fuel dispensing installation (17) for supplying vehicles with hydrogen generated by the hydrogen generating device and/or electrical energy generated by the regenerative electricity generating device and/or supplementary or replacement gas provided by the gas providing device.

6. The energy supply system as claimed in any of Claims 2 to 5, **characterized by** an electricity supply grid (2) to which the regenerative electricity generating device, the hydrogen generating device, and the gas-into-electricity conversion device are connected, and/or a gas supply grid (7) to which the gas providing device and the gas-into-electricity conversion device are connected.

7. The energy supply system as claimed in Claim 6, **characterized by** a gas storage (8) which is connected to the gas supply grid.

8. The energy supply system as claimed in Claim 6 or 7, **characterized by** a control device which is configured to variably set the power need of the hydrogen generating device and/or the feed-in power of the gas-into-electricity conversion device depending on a time-dependent power demand on the electricity grid.

9. The energy supply system as claimed in Claim 8, **characterized in that**
- the control device is configured to set a reduced power need of the hydrogen generating device and/or an increased feed-in power of the gas-into-electricity conversion device at periods when there is an increased demand on the electricity grid for power generation, and/or to set an increased power need of the hydrogen generating device and/or a reduced feed-in power of the gas-into-electricity conversion device at periods when there is a reduced demand on the electricity grid for power generation, and/or
- the control device is configured to set the power need of the hydrogen generating device and/or the feed-in power of the gas-into-electricity conversion device depending on a projected power demand profile of the electricity supply grid and/or a projected electricity generating power profile of the regenerative electricity generating device.

10. The energy supply system as claimed in any of Claims 1 to 9, **characterized in that** the gas providing device is configured to provide the supplementary gas or replacement gas selectively in one of a plurality of predetermined quality levels.

11. A method for operating an energy supply system according to any of Claims 1 to 10, wherein the power need of the hydrogen generating device and/or the feed-in power of the gas-into-electricity conversion device is variably set depending on a time-dependent power demand on the electricity supply grid, and/or wherein the supplementary gas or replacement gas is provided in a variably specifiable supplementary/replacement gas quality suitable for feeding into a gas supply grid using the gas containing methane from the methanation device and the hydrogen from the hydrogen generating device depending on the determined or supplied base gas composition in the gas grid.

12. The method as claimed in Claim 11, **characterized in that** a reduced power consumption of the hydrogen generating device and/or an increased feed-in power of the gas-into-electricity conversion device is set at periods when there is an increased demand on the electricity grid for power, and/or an increased power consumption of the hydrogen generating device and/or a reduced feed-in power of the gas-into-electricity conversion device at periods is set when there is a reduced demand on the electricity grid for power.

13. The method as claimed in Claim 11 or 12, **characterized in that** the power consumption of the hydrogen generating device and/or the feed-in power of the gas-into-electricity conversion device is set depending on a projected power demand profile of the electricity supply grid and/or a projected electricity generating power profile of the electricity generating device.

14. The method as claimed in any of Claims 11 to 13, **characterized in that** the supplementary or replacement gas is provided selectively in one of a plurality of predetermined quality levels.

## Revendications

1. Système d'approvisionnement en énergie, avec :
- un dispositif de production d'électricité (1) pour la production renouvelable d'énergie électrique pouvant alimenter un réseau d'alimentation électrique (2),
- un dispositif de production d'hydrogène (3) pour la production d'hydrogène en utilisant de l'énergie électrique du dispositif de production d'électricité renouvelable,
- un dispositif de méthanisation (4) pour la transformation d'hydrogène produit par le dispositif de production d'hydrogène et d'un gaz d'oxyde de carbone amené en un gaz à teneur en méthane, et
- un dispositif de mise à disposition de gaz (11) avec des moyens de préparation de gaz pour la préparation et la mise à disposition d'un gaz additionnel ou d'un gaz de remplacement d'une qualité de gaz additionnel/de remplacement prescriptible de façon variable, qui convient pour l'alimentation d'un réseau d'alimentation en gaz, en utilisant des parts variables de manière correspondante du gaz à teneur en méthane provenant du dispositif de méthanisation et de l'hydrogène provenant du dispositif de production d'hydrogène et avec des moyens de commande associés, lesquels sont étudiés pour reconnaître la composition d'un gaz de base déjà présent dans le réseau d'alimentation en gaz ou pour réceptionner une information amenée à ce sujet et en fonction de cela, pour décider de la qualité de gaz d'addition/de remplacement à régler et pour commander de façon variable l'ajout par mélange des différents constituants du gaz pour la préparation du gaz d'addition ou de remplacement selon la qualité de gaz concernée.

2. Système d'approvisionnement en énergie selon la revendication 1, **caractérisé par** une centrale de production électrique (10) pour la production d'énergie électrique en utilisant du gaz provenant d'un réseau d'alimentation en gaz.

3. Système d'approvisionnement en énergie selon la revendication 1 ou 2, **caractérisé en ce que** le dispositif de production d'électricité renouvelable comprend une ou plusieurs éoliennes (la) et/ou une ou plusieurs installations photovoltaïques (1b) et/ou une ou plusieurs centrales géothermiques et/ou une ou plusieurs centrales à biomasse et/ou une ou plusieurs centrales hydroélectriques et/ou une ou plusieurs centrales thermiques solaires.

4. Système d'approvisionnement en énergie selon l'une des revendications 1 à 3, **caractérisé par**
- des moyens pour l'amenée de dioxyde de carbone au dispositif de méthanisation à partir d'une centrale électrique émettant du dioxyde de carbone, et/ou
- des moyens pour l'amenée d'un gaz à teneur en dioxyde de carbone au dispositif de méthanisation à partir d'une centrale à biomasse, et/ou
- des moyens pour l'amenée d'un gaz de synthèse à teneur en oxyde de carbone, à partir d'une installation de gazéification de biomasse, au dispositif de méthanisation, et/ou
- des moyens pour le transfert de chaleur du dispositif de méthanisation à l'installation de biogaz, et/ou
- des moyens pour l'amenée d'un gaz additif ou de remplacement, obtenu de la part de l'installation de biogaz, au dispositif de mise à disposition de gaz ou au réseau d'alimentation en gaz.

5. Système d'approvisionnement en énergie selon l'une des revendications 1 à 4, **caractérisé par**
- une installation ORC (13) ou une autre installation d'utilisation de chaleur pour la production d'énergie électrique en utilisant la chaleur perdue du dispositif de méthanisation, et/ou
- un dispositif de station-service (17) pour alimenter des véhicules avec de l'hydrogène produit par le dispositif de production d'hydrogène et/ou avec de l'énergie électrique produite par le dispositif de production d'électricité renouvelable et/ou avec du gaz additionnel ou de remplacement mis à disposition par le dispositif de mise à disposition de gaz.

6. Système d'approvisionnement en énergie selon l'une des revendications 2 à 5, **caractérisé par** un réseau d'alimentation électrique (2) auquel sont raccordés le dispositif de production d'électricité renouvelable, le dispositif de production d'hydrogène et la centrale de production électrique, et/ou un réseau d'alimentation en gaz (7) auquel sont raccordés le dispositif de mise à disposition de gaz et la centrale de production électrique.

7. Système d'approvisionnement en énergie selon la revendication 6, **caractérisé par** un accumulateur de gaz (8) qui est raccordé au réseau d'alimentation en gaz.

8. Système d'approvisionnement en énergie selon la revendication 6 ou 7, **caractérisé par** un dispositif de commande, lequel est étudié pour régler de façon variable la puissance de consommation du dispositif de production d'hydrogène et/ou la puissance d'alimentation de la centrale de production électrique en fonction d'un besoin en énergie en fonction du temps du réseau électrique.

9. Système d'approvisionnement en énergie selon la revendication 8, **caractérisé en ce que**
- le dispositif de commande est étudié pour régler, dans des intervalles de temps à besoin en énergie de production accru du réseau d'alimentation électrique, une puissance de prélèvement réduite du dispositif de production d'hydrogène et/ou une puissance d'alimentation accrue de la centrale de production électrique, et/ou pour régler, dans des intervalles de temps à besoin en énergie de production réduit du réseau d'alimentation électrique, une puissance de prélèvement accrue du dispositif de production d'hydrogène et/ou une puissance d'alimentation réduite de la centrale de production électrique, et/ou
- **en ce que** le dispositif de commande est étudié pour régler la puissance de prélèvement du dispositif de production d'hydrogène et/ou la puissance d'alimentation de la centrale de production électrique en fonction d'un profil de besoin en énergie pronostiqué du réseau d'alimentation électrique et/ou d'un profil de puissance de production électrique pronostiqué du dispositif de production d'électricité renouvelable.

10. Système d'approvisionnement en énergie selon l'une des revendications 1 à 9, **caractérisé en ce que** le dispositif de mise à disposition de gaz est étudié pour mettre à disposition le gaz additionnel ou de remplacement, au choix, dans un niveau de qualité parmi plusieurs niveaux de qualité prescriptibles.

11. Procédé pour exploiter un système d'approvisionnement en énergie selon l'une des revendications 1 à 10, dans lequel la puissance de prélèvement du dispositif de production d'hydrogène et/ou la puissance d'alimentation de la centrale de production électrique est réglée de façon variable en fonction d'un besoin en énergie en fonction du temps du réseau d'alimentation électrique, et/ou dans lequel le gaz additionnel ou le gaz de remplacement est mis à disposition avec une qualité de gaz additionnel/de remplacement prescriptible de façon variable, laquelle convient pour l'alimentation d'un réseau d'alimentation en gaz en utilisant le gaz à teneur en méthane provenant du dispositif de méthanisation et l'hydrogène provenant du dispositif de production d'hydrogène en fonction de la composition de gaz de base reconnue ou amenée dans le réseau d'alimentation en gaz.

12. Procédé selon la revendication 11, **caractérisé en ce que**, dans des intervalles de temps à besoin en énergie accru du réseau d'alimentation électrique, on règle une puissance de prélèvement réduite du dispositif de production d'hydrogène et/ou une puissance d'alimentation accrue de la centrale de production électrique, et/ou **en ce que**, dans des intervalles de temps à besoin en énergie réduit du réseau d'alimentation électrique, on règle une puissance de prélèvement accrue du dispositif de production d'hydrogène et/ou une puissance d'alimentation réduite de la centrale de production électrique.

13. Procédé selon la revendication 11 ou 12, **caractérisé en ce que** la puissance de prélèvement du dispositif de production d'hydrogène et/ou la puissance d'alimentation de la centrale de production électrique est réglée en fonction d'un profil de besoin en énergie pronostiqué du réseau d'alimentation électrique et/ou d'un profil de puissance de production électrique pronostiqué du dispositif de production d'électricité.

14. Procédé selon l'une des revendications 11 à 13, **caractérisé en ce que** le gaz additionnel ou de remplacement est mis à disposition, au choix, avec un niveau de qualité parmi plusieurs niveaux de qualité prescriptibles.
